# EUROPEAN PATENT SPECIFICATION

(11) **EP 0 912 191 B1**
(45) Date of publication and mention of the grant of the patent: **09.04.2003**
(21) Application number: 98914147.8
(22) Date of filing: 16.04.1998
(51) Int. Cl.: A61K 35/78

(54) **VEGETABLE EXTRACT, METHOD FOR PREPARATION THEREOF AND ITS APPLICATIONS IN HUMAN AND VETERINARY MEDICINE**
PFLANZENEXTRAKT, VERFAHREN ZU DESSEN HERSTELLUNG UND SEINE VERWENDUNGEN IN DER HUMAN- UND TIERMEDIZIN
EXTRAIT VEGETAL, PROCEDE DE PREPARATION ASSOCIE, ET UTILISATIONS EN MEDECINE HUMAINE ET VETERINAIRE

(30) Priority: 16.04.1997 NL 1005821
(43) Date of publication of application: 06.05.1999
(73) Proprietor: Feva N.V., Curacao (AN); Gillessen, Hubert Jean Marie Francois, 3620 Lanaken (BE)
(72) Inventor: GILLESSEN, Hubert, Jean, Marie, François, B-3620 Lanaken (BE)
(74) Representative: Van Someren, Petronella F. H. M.
(86) International application number: NL9800214
(87) International publication number: WO98046244

(56) References cited:
- WO-A-92/00085
- FR-A- 2 732 347
- R. ADETORO TOGUN ET AL.: "A GALACTOSE-BINDING T-CELL MITOGENIC LECTIN FROM THE SEEDS OF TELFAIRIA OCCIDENTALIS" PHYTOCHEMISTRY, vol. 35, no. 5, 1994, pages 1125-1130, XP002050490

## Description

The present invention relates to new vegetable extracts, to their method of preparation and to their application in biotechnology and therapy, in particular due to their activity as cell growth- and growth-inhibiting factor.

Plants often possess unsuspected therapeutic properties or constituents active as such. Particularly for the treatment of disorders which are difficult to treat, such as cancer and the like, plants are a potential source of medicines.

According to the present invention it has now been found, unexpectedly, that extracts which are biologically active can be prepared from plants, in particular vegetables containing glucide, protein, glycoprotein and terpene. For this purpose can be used vegetables such as potatoes, radish, turnip, beet, carrot, Jerusalem artichoke, sweet potato, rape, cucumber, pumpkin, courgette, tomato and the like. This list is not exhaustive.

The extracts according to the invention are prepared in accordance with the following successive phases of:
- pressing the chosen vegetables to pulp;
- cold or hot treatment of the obtained pulp with an aqueous or organic solvent;
- evaporating the solvent until a determined degree of concentration is obtained;
- optional further purifying of the thus obtained extract by one or more of the following methods such as thermocoagulation, purification with ammonium sulphate, precipitation, chromatography on gel or ion-exchange resin.

A method of extraction which makes it possible to obtain three fractions having a determined activity has already been described in the French patent application FR-A-2 732 347 in the name of the company BIO MEDIA.

According to the invention the extracts are purified still further, which has resulted in 16 molecules of the type glucide, protein, glycoprotein, terpene and peptide with molecular weights of 5,000, 7,500, 10,000, 12,000, 15,000, 20,000, 30,000, 40,000, 65,000, 82,000, 105,000, 130,000, 180,000, 200,000, 220,000, 250,000 daltons. All these molecules are readily soluble in water.

These molecules can be obtained by further purifying the extract by chromatography on Q hyper DTM20-gel. 11 molecules are hereby obtained which elute at 0.0-9 M, 0.15 M, 0.21 M, 0.25 M, 0.32 M, 0.45 M. 0.52 M, 0.70 M, 0.78 M, 0.85 M, 1.02 M NaCl.

In the eluate of the column are situated the low-molecular constituents which can be further purified by chromatography on a second column based on QMA-gel. Five fractions were eluted at 0.05 M, 0.08 M, 0.12 M, 0.20 M and 0.27 M NaCl.

Of the 16 different constituents obtained in this manner, the activity was determined as described in the examples.

The toxicological and pharmacological studies described in the examples show the absence of toxicity of the compounds according to the invention, as well as their significant properties.

The 14 molecules with mitogenic activity according to the invention can each be used as such or in different concentrations for different purposes, such as the manufacture of culture media for research and diagnosis, for the production of molecules and vaccines for therapeutic use for human or animal medicine, or for cosmetic use.

The five molecules which stimulate multiplication of micro-organisms can advantageously be used in the field of fermentation culture media, for the production of micro-organisms or of molecules for human and animal therapeutics, cosmetics or the agribusiness.

The three molecules which have an effect on the metabolism are to be advantageously administered in human and animal medicine in the case of weight deficiency or growth problems, or for bacteriological diagnostics.

The two molecules, which have a cell-inhibiting activity, are to be advantageously administered as antitumour agents and anti-cancer agents due to their cytostatic capacity.

The invention will be further illustrated in the following non-limiting examples.

### EXAMPLES

### EXAMPLE 1

### Isolation of active substances

1000 grams of a fresh, healthy pumpkin is crushed in order to obtain a fine puree. The puree obtained in this manner is treated for two hours with 500 grams of cold water. The paste (E1) obtained is then either centrifuged or filtered to collect the pulp (E2).

The thus obtained extract is then purified by thermocoagulation in the presence of alcohol, caprylic acid, acetic acid and ammonium sulphate. After centrifugation the supernatant is recovered, and subsequently subjected to ultrafiltration in order to eliminate particular salts and to obtain the correct concentration of filtrate.

With chromatographic purification on Q hyper DTM20-gel of the thus obtained concentrate, 11 molecules are obtained which elute at 0.09 M, 0.15 M, 0.21 M, 0.25 M, 0.32 M, 0.45 M, 0.52 M, 0.70 M, 0.78 M, 0.85 M, 1.02 M NaCl. Reference will be made in the following to the different fractions on the basis of the concentration at which they elute.

The lower molecular weights are situated in the eluate when the column is loaded. They are purified on a second column on QMA-gel and eluted at 0.05 M, 0.08 M, 0.12 M, 0.20 M and 0.27 M NaCl.

### EXAMPLE 2

### Activity

The molecules isolated in Example 1 exhibit a high mitogenic capacity for all animal or human cells, with the exception of the molecules which are isolated at 0.32 M on Q hyper DTM20-gel and at 0.12 M on QMA-gel, which have a significant cell growth-inhibiting activity.

### EXAMPLE 3

### Toxicological study

Tests performed both in vivo and in vitro show the low toxicity and good tolerance of the active ingredients according to the invention.

The cellular toxicity in vitro was determined using neutral red and MTT. At a dose of 600 µg/ml no toxicity was detected in the culture medium.

The following in vivo tests were carried out. All molecules displaying mitogenic activities were tested on mice. This study demonstrated the absence of toxicity. The intraperitoneal injection of 0.5 ml of a 100 g/l solution of each of these molecules on two groups of 24 male animals and 24 female animals caused no mortality after seven days.

The same tests were performed with the same doses using the molecules which inhibit the cell growth. At the beginning of the experiment an average weight loss of 3-4% in the treated animals was observed. This was the case for both the females and the males. Four to five days after injection the animals resumed their normal growth, which demonstrates the non-toxicity of the molecules according to the invention.

### EXAMPLE 4

### Pharmacological study

Results of the pharmacological tests reported here show the significant properties of the active ingredient according to the invention.

### 1. Mitogenic activity

The tests were performed on stocks of CRFK- and VERO-cells while making use of NUNC-plates with 6 wells and HAM F12 medium.

The medium was strewn with 200,000 cells/ml. Each molecule was tested in cell culture relative to the controls consisting of only HAM F12 and HAM F12 + 10% foetal calf serum (FCS) in doses varying from 0.025 g/l to 4 g/l.

For each molecule the best concentration range was determined in respect of activity. The average activities lie between 0.05 g/l and 0.05 g/l.

The detected rate of multiplication of the cells varies between 3 and 5, depending on the tested molecule, i.e. at a starting quantity of 200,000 cells/ml, 600,000 to 1,000,000 cells/ml are formed. The rate of multiplication is comparable with the values obtained with the SVF-cells: 900,000 to 1,000,000 cells/ml.

The mitogenic capacity of the molecules according to the invention was subsequently tested on more than 120 cell lines referred to in the ATCC, or in primo-culture, animal, eukaryotic, human or diploid cells.

In addition to their activity on the cell lines, it was observed that the molecules which eluted at 0.09 M, 0.15 M, 0.70 M, 0.85 M and 1.02 M likewise exhibit a considerable multiplying capacity on micro-organisms. Tests performed on Lactobacillus thus showed an increase in biomass of 30% in 24 hours relative to the control.

### 2. Inhibiting activity

In tests performed under the same conditions, the molecules which eluted at 0.32 M on the Q Hyper DTM 20-column and 0.12 M on the QMA-column produced a total inhibition of the cell growth at doses between 0.5 g/l and 1 g/l.

These two molecules likewise inhibited the growth of the complete panel of tumour cells which is required by the NCI (62 stocks).

### 3. Effect on the metabolism in vivo

This study was carried out with the molecules which eluted at 0.21 M, 0.45 M on the Q Hyper DTM 20-column and at 0.20 M on the QMA-column. Different groups of 24 male mice and 24 female mice received 0.5 ml of a 100 g/l solution of the molecule for testing.

The molecules of the invention caused an average weight increase of 8,25% in both the male and female mice relative to the control groups, which only received an injection of 0.5 ml of a 100 g/l solution of sodium chloride.

## Claims

1. Biologically active compound obtainable by a method comprising the following steps of:
a) pressing a vegetable material to pulp;
b) subjecting the obtained pulp to a cold or hot treatment with an aqueous or organic solvent;
c) removing the solvent until a determined degree of concentration is obtained; and
d) purifying of the thus obtained extract by one or more of the following methods: thermocoagulation, purification with ammonium sulphate, precipitation, chromatography on gel or ion-exchange resin;
e) further purifying of the extract on a Q hyper DTM20-gel chromatography column, eluting the column and subsequently collecting fractions which elute at determined concentrations of the eluent, **characterized in that** the biologically active compound elutes at 0.09 M NaCl, 0.15 M NaCl, 0.21 M NaCl, 0.25 M NaCl, 0.32 M NaCl, 0.45 M NaCl, 0.52 M NaCl, 0.70 M NaCl, 0.78 M NaCl, 0.85 M NaCl, or 1.02 M NaCl.

2. Biologically active compound as claimed in claim 1 **characterized in that** the method further comprises step
f) further purifying the eluate of step e) on a QMA-gel chromatography column, eluting the column and subsequently collecting fractions which elute at determined concentrations of the eluent, wherein the compound elutes at 0.05 M NaCl, 0.08 M NaCl, 0.12 M NaCl, 0.20 M NaCl, or 0.27 M NaCl.

3. Biologically active compound as claimed in claim 1 or 2, which is obtained from a vegetable chosen from potatoes, radish, turnip, beet, carrot, Jerusalem artichoke, sweet potato, rape, cucumber, pumpkin, courgette and tomato.

4. Biologically active compound as claimed in claim 1 which elutes at 0.09 M NaCl, 0.15 M NaCl 0.21 M NaCl, 0.25 M NaCl, 0.45 M NaCl, 0.52 M NaCl, 0.70 M NaCl, 0.78 M NaCL, 0.85 M or 1.02 M NaCl or as claimed in claim 2 which elutes at 0.05 M NaCl, 0.08 M NaCl, 0.20 M NaCl, or 0.27 M NaCl, or combinations thereof, for use as a mitogen for eukaryotic cells.

5. Biologically active compound as claimed in claim 1 which elutes at 0.32 M NaCL or as claimed in claim 2 which elutes at 0.12 M NaCl, or a combination thereof, for use as cell growth-inhibiting agent.

6. Biologically active compound as claimed in claim 1 which elutes at 0.21 M NaCl or 0.45 M NaCl, or as claimed in claim 2 which elutes at 0.20 M, for use as agent for stimulating the metabolism.

7. Biologically active compound as claimed in claim 1 which elutes at 0.09 M NaCl, 0.15 M NaCl, 0.70 M NaCl, 0.85 M NaCl, or 1.02 M NaCl, or combinations thereof, for use as mitogen for micro-organisms.

8. Method for manufacturing a biologically active compound with a biological activity which consists of influencing cell growth, wherein the method comprises of:
a) pressing a vegetable material to pulp;
b) subjecting the obtained pulp to a cold or hot treatment with an aqueous or organic solvent;
c) removing the solvent until a determined degree of concentration is obtained;
d) purifying of the thus obtained extract by one or more of the following methods: thermocoagulation, purification with ammonium sulphate, precipitation, chromatography on gel or ion-exchange resin;
e) subjecting the extract to a chromatographic separation on a Q hyper DTM20-gel and eluting the column with a gradient of NaCl; and
f) optionally further subjecting the eluate of step e) on a column with QMA-gel and eluting of the column with a gradient of NaCl.

9. Method as claimed in claim 8, **characterized in that**
a) the treatment of the obtained pulp is carried out with cold water,
b) the thus obtained material is concentrated by centrifugation or filtration,
c) a thermocoagulation is subsequently performed in the presence of alcohol, caprylic acid, acetic acid and ammonium sulphate,
d) the thus obtained extract is centrifuged,
e) the supernatant is subjected to ultrafiltration to obtain the extract.

10. Medication for human or veterinary use, comprising at least one of the biologically active compounds as claimed in claims 1-7.

11. Culture medium, comprising at least one of the biologically active compounds as claimed in claims 1-7.

## Patentansprüche

1. Biologisch aktive Verbindung, erhältlich mit einem Verfahren, das die folgenden Schritte umfaßt:
a) Pressen eines Pflanzenmaterials zu Brei;
b) Unterwerfen des erhaltenen Breis unter eine kalte oder heiße Behandlung mit einem wäßrigen oder organischen Lösungsmittel;
c) Entfernen des Lösungsmittels, bis ein festgelegter Konzentrationsgrad erreicht ist; und
d) Reinigen des so erhaltenen Extraktes mit einem oder mehreren der folgenden Verfahren: Thermokoagulation, Reinigung mit Ammoniumsulfat, Ausfällung, Chromatographie auf Gel oder Ionenaustauschharz;
e) weiteres Reinigen des Extraktes auf einer Q-Hyper-DTM20-Gelchromatographiesäule, Eluieren der Säule und anschließend Sammeln von Fraktionen, die bei bestimmten Konzentrationen des Elutionsmittels eluieren, **dadurch gekennzeichnet, daß** die biologisch aktive Verbindung bei 0,09 M NaCl, 0,15 M NaCl, 0,21 M NaCl, 0,25 M NaCl, 0,32 M NaCl, 0,45 M NaCl, 0,52 M NaCl, 0,70 M NaCl, 0,78 M NaCl, 0,85 M NaCl oder 1,02 M NaCl eluiert.

2. Biologisch aktive Verbindung nach Anspruch 1, **dadurch gekennzeichnet, daß** das Verfahren weiter den Schritt umfaßt:
f) weiteres Reinigen des Eluats aus Schritt e) auf einer QMA-Gelchromatographiesäule, Eluieren der Säule und anschließend Sammeln von Fraktionen, die bei bestimmten Konzentrationen des Elutionsmittels eluieren, wobei die Verbindung bei 0,05 M NaCl, 0,08 M NaCl, 0,12 M NaCl, 0,20 M NaCl oder 0,27 M NaCl eluiert.

3. Biologisch aktive Verbindung nach Anspruch 1 oder 2, die erhalten wird aus einer Pflanze, die ausgewählt ist aus Kartoffeln, Rettich, Rübe, Bete, Karotte, Jerusalem-Artischocke, Süßkartoffel, Raps, Gurke, Kürbis, Zucchini und Tomate.

4. Biologisch aktive Verbindung nach Anspruch 1, die bei 0,09 M NaCl, 0,15 M NaCl, 0,21 M NaCl, 0,25 M NaCl, 0,45 M NaCl, 0,52 M NaCl, 0,70 M NaCl, 0,78 M NaCl, 0,85 M NaCl oder 1,02 M NaCl eluiert, oder nach Anspruch 2, die bei 0,05 M NaCl, 0,08 M NaCl, 0,20 M NaCl oder 0,27 M NaCl eluiert, oder Kombinationen davon, zur Verwendung als ein Mitogen für eukaryontische Zellen.

5. Biologisch aktive Verbindung nach Anspruch 1, die bei 0,32 M NaCl eluiert, oder nach Anspruch 2, die bei 0,12 M NaCl eluiert, oder einer Kombination davon, zur Verwendung als zellwachstumshemmendes Mittel.

6. Biologisch aktive Verbindung nach Anspruch 1, die bei 0,21 M NaCl oder 0,45 M NaCl eluiert, oder nach Anspruch 2, die bei 0,20 M eluiert, zur Verwendung als Mittel zur Stimulierung des Stoffwechsels.

7. Biologisch aktive Verbindung nach Anspruch 1, die bei 0,09 M NaCl, 0,15 M NaCl, 0,70 M NaCl, 0,85 M NaCl oder 1,02 M NaCl eluiert, oder Kombinationen davon, zur Verwendung als Mitogen für Mikroorganismen.

8. Verfahren zur Herstellung einer biologisch aktiven Verbindung mit einer biologischen Aktivität, die aus der Beeinflussung des Zellwachstums besteht, wobei das Verfahren umfaßt:
a) Pressen eines Pflanzenmaterials zu Brei;
b) Unterwerfen des erhaltenen Breis unter eine kalte oder heiße Behandlung mit einem wäßrigen oder organischen Lösungsmittel;
c) Entfernen des Lösungsmittels, bis ein festgelegter Konzentrationsgrad erreicht ist;
d) Reinigen des so erhaltenen Extraktes mit einem oder mehreren der folgenden Verfahren: Thermokoagulation, Reinigung mit Ammoniumsulfat, Ausfällung, Chromatographie auf Gel oder Ionenaustauschharz;
e) Unterwerfen des Extraktes unter eine chromatographische Trennung auf einem Q-Hyper-DTM20-Gel und Eluieren der Säule mit einem NaCl-Gradienten; und
f) fakultativ weiteres Unterwerfen des Eluats aus Schritt e) auf einer Säule mit QMA-Gel und Eluieren der Säule mit einem NaCl-Gradienten.

9. Verfahren nach Anspruch 8, **dadurch gekennzeichnet, daß**
a) die Behandlung des erhaltenen Breis mit kaltem Wasser durchgeführt wird,
b) das so erhaltene Material durch Zentrifugation oder Filtration konzentriert wird,
c) eine Thermokoagulation anschließend in Gegenwart von Alkohol, Caprylsäure, Essigsäure und Ammoniumsulfat durchgeführt wird,
d) der so erhaltene Extrakt zentrifugiert wird,
e) der Überstand einer Ultrafiltration unterworfen wird, um den Extrakt zu erhalten.

10. Medikament für Human- oder Veterinärgebrauch, das wenigstens eine der biologisch aktiven Verbindungen nach den Ansprüchen 1 bis 7 umfaßt.

11. Kulturmedium, das wenigstens eine der biologisch aktiven Verbindungen nach den Ansprüchen 1 bis 7 umfaßt.

## Revendications

1. Composé biologiquement actif qui peut être obtenu par un procédé qui comporte les étapes suivantes :
a) le pressage d'un matériau végétal sous forme d'une pulpe,
b) l'application à la pulpe obtenue d'un traitement par le froid ou la chaleur à l'aide d'un solvant aqueux ou organique,
c) l'enlèvement du solvant jusqu'à l'obtention d'un degré déterminé de concentration, et
d) la purification de l'extrait ainsi obtenu à l'aide d'un ou plusieurs des procédés suivantes : la thermocoagulation, la purification par le sulfate d'ammonium, la précipitation, la chromatographie sur gel ou sur résine échangeuse d'ions,
e) la purification supplémentaire de l'extrait sur une colonne de chromatographie sur gel "Q hyper DTM20-gel", l'élution de la colonne, puis la collecte des fractions éluées à des concentrations déterminées de l'éluant, **caractérisé en ce que** le composé biologiquement actif est élué à 0,09 M de NaCl, 0,15 M de NaCl, 0,21 M de NaCl, 0,25 M de NaCl, 0,32 M de NaCl, 0,45 M de NaCl, 0,52 M de NaCl, 0,70 M de NaCl, 0,78 M de NaCl, 0,85 M de NaCl ou 1,02 M de NaCl.

2. Composé biologiquement actif selon la revendication 1, **caractérisé en ce que** le procédé comporte en outre l'étape suivante :
f) la purification supplémentaire de l'éluant de l'étape e) sur une colonne de chromatographie sur gel "QMA-gel", l'élution dans la colonne, puis la collecte des fractions éluées à des concentrations déterminées de l'éluant, telles que le composé est élué à 0,05 M de NaCl, 0,08 M de NaCl, 0,12 M de NaCl, 0,20 M de NaCl ou 0,27 M de NaCl.

3. Composé biologiquement actif selon la revendication 1 ou 2, qui est obtenu à partir d'un légume choisi parmi la pomme de terre, le radis, le navet, la betterave, la carotte, l'artichaut de Jérusalem, la patate douce, la navette, le concombre, le potiron, la courgette et la tomate.

4. Composé biologiquement actif qui est élué à 0,09 M de NaCl, 0,15 M de NaCl, 0,21 M de NaCl, 0,25 M de NaCl, 0,45 M de NaCl, 0,52 M de NaCl, 0,70 M de NaCl, 0,78 M de NaCl, 0,85 M de NaCl ou 1,02 M de NaCl ou selon la revendication 2 qui est élué à 0,05 M de NaCl, 0,08 M de NaCl, 0,20 M de NaCl ou 0,27 M de NaCl ou leurs combinaisons, destiné à être utilisé comme agent mitogène pour les cellules eucaryotes.

5. Composé biologiquement actif selon la revendication 1, qui est élué à 0,32 M de NaCl, ou selon la revendication 2 qui est élué à 0,12 M de NaCl, ou une de leurs combinaisons, destiné à être utilisé comme agent d'inhibition de croissance cellulaire.

6. Composé biologiquement actif selon la revendication 1, qui est élué à 0,21 M de NaCl ou 0,45 M de NaCl ou selon la revendication 2 qui est élué à 0,20 M de NaCl, destiné à être utilisé comme agent de stimulation du métabolisme.

7. Composé biologiquement actif selon la revendication 1, qui est élué à 0,09 M de NaCl, 0,15 M de NaCl, 0,70 M de NaCl, 0,85 M de NaCl ou 1,02 M de NaCl, ou une de leurs combinaisons, destiné à être utilisé comme agent mitogène pour des microorganismes.

8. Procédé de fabrication d'un composé biologiquement actif ayant une activité biologique, qui consiste en une influence sur la croissance cellulaire, dans lequel le procédé comprend :
a) le pressage d'un matériau à base de légumes sous forme d'une pulpe,
b) l'application à la pulpe obtenue d'un traitement par le froid ou par le chaud à l'aide d'un solvant aqueux ou organique,
c) l'enlèvement du solvant jusqu'à l'obtention d'un degré déterminé de concentration,
d) la purification de l'extrait ainsi obtenu par un ou plusieurs des procédés suivants : la thermocoagulation, la purification par le sulfate d'ammonium, la précipitation, la chromatographie sur gel ou sur résine d'échange d'ions,
e) l'application à l'extrait d'une séparation chromatographique sur gel "Q hyper DTM20-gel" et l'élution dans la colonne avec un gradient de NaCl, et
f) éventuellement un traitement supplémentaire de l'éluant de l'étape e) sur une colonne avec un gel "QMA-gel" et l'élution dans la colonne avec un gradient de NaCl.

9. Procédé selon la revendication 8, **caractérisé en ce que** :
a) le traitement de la pulpe obtenue est exécuté avec de l'eau froide,
b) le matériau ainsi obtenu est concentré par centrifugation ou filtration,
c) une thermocoagulation est ensuite exécutée en présence d'un alcool, d'acide caprylique, d'acide acétique et de sulfate d'ammonium,
d) l'extrait ainsi obtenu est centrifugé, et
e) la matière qui surnage est soumise à une ultrafiltration pour l'obtention de l'extrait.

10. Agent médicamenteux pour utilisation humaine ou vétérinaire, comprenant au moins l'un des composés biologiquement actifs selon les revendications 1 à 7.

11. Milieu de culture, comprenant au moins l'un des composés biologiquement actifs selon les revendications 1 à 7.
